# EUROPÄISCHE PATENTSCHRIFT

(11) **EP 1 088 224 B1**
(45) Veröffentlichungstag und Bekanntmachung des Hinweises auf die Patenterteilung: **02.01.2002**
(21) Anmeldenummer: 99938135.3
(22) Anmeldetag: 07.06.1999
(51) Int. Cl.: G01N 33/28, G01N 1/34

(54) **GASEXTRAKTIONSANLAGE**
GAS EXTRACTION FACILITY
INSTALLATION D'EXTRACTION DE GAZ

(30) Priorität: 19.06.1998 DE 19827444
(43) Veröffentlichungstag der Anmeldung: 04.04.2001
(73) Patentinhaber: SIEMENS AKTIENGESELLSCHAFT, 80333 München (DE)
(72) Erfinder: LÜTKE, Harry, D-90461 Nürnberg (DE); KUSCHEW, Irin, D-90522 Oberasbach (DE); NEUBEHLER, Heinz, D-90471 Nürnberg (DE)
(86) Internationale Anmeldenummer: DE9901658
(87) Internationale Veröffentlichungsnummer: WO9967633

(56) Entgegenhaltungen:
- GB-A- 1 397 935
- GB-A- 2 188 437
- US-A- 4 236 404
- US-A- 4 409 814
- US-A- 5 062 292
- US-A- 5 659 126
- INOUE Y ET AL: "DEVELOPMENT OF OIL-DISSOLVED HYDROGEN GAS DETECTOR FOR DIAGNOSIS OFTRANSFORMERS" IEEE TRANSACTIONS ON POWER DELIVERY, Bd. 5, Nr. 1, 1. Januar 1990 (1990-01-01), Seiten 226-232, XP000086404 ISSN: 0885-8977
- IEC: "International Standard IEC 567: guide for the sampling of gases and of oil from oil-filled electrical equipment and for the analysis of free and dissolved gases" Juli 1992 (1992-07) , IEC , GENEVE XP000852699 in der Anmeldung erwähnt Absatz [07.2]; Abbildung 9

## Beschreibung

Die Erfindung betrifft eine Gasextraktionsanlage, insbesondere für eine Gas-In-Öl-Analyse, mit einem Entgasungsrezipienten, der eine Einfüllvorrichtung für ein zu entgasendes Medium aufweist und der über ein Ventil mit einem außerhalb des Entgasungsrezipienten angeordneten zylindrisch ausgebildeten Gassammelraum verbindbar ist, dessen Volumen mittels eines verschiebbaren Kolbens veränderbar ist und der über eine Gasleitung mit einem Analysegerät verbunden ist.

Eine solche Gasextraktionsanlage ist aus der US-PS 4,409,814 bekannt. Dort ist ein Entgasungsrezipient vorgesehen, der eine nicht näher dargestellte Einfüllvorrichtung für zu entgasendes Öl aufweist und der über ein Ventil mit einem außerhalb des Entgasungsrezipienten angeordneten zylindrisch ausgebildeten Gassammelraum verbindbar ist, dessen Volumen mittels eines verschiebbaren Kolbens veränderbar ist. Der Gassammelraum befindet sich innerhalb eines Zylinders, in dem der Kolben verschiebbar angeordnet ist. An den Zylinder schließt sich ein weiterer Zylinder mit einem größeren Durchmesser als der Zylinder an, in dem ein weiterer verschiebbarer Kolben angeordnet ist, der über eine Stange mit dem Kolben verbunden ist. Der weitere Zylinder und der Kolben dienen dazu, den Kolben mehrfach im Zylinder hin- und herschieben zu können. Der Zylinder bildet mit dem Kolben dadurch eine Art Pumpe, bei der der Gassammelraum als Raum für ein zu pumpendes Medium, in diesem Fall also das extrahierte Gas dient. Zur Aufnabme des extrahierten Gases ist zusätzlich ein Gassammelrohr vorgesehen, das über ein Ventil mit dem Gassammelraum verbunden ist. Zu Beginn eines Entgasungsvorgangs befindet sich der Kolben zunächst in einer solchen Position, dass das Volumen des Gassammelraums maximal ist. Der Gassammelraum ist über das Ventil mit dem Entgasungsrezipienten verbunden. Zunächst werden der Entgasungsrezipient, der Gassammelraum und das Gassammelrohr mittels einer Vakuumpumpe evakuiert. In den Entgasungsrezipienten wird danach die zu entgasende Ölprobe eingebracht. Mittels einer Magnetrühreinrichtung wird das im Entgasungsrezipienten befindliche Öl gerührt, um die Entgasung zu unterstützen. Das aus dem Öl ausgetretene Gas tritt über das Ventil in den Gassammelraum ein. Um eine Rückdiffusion von extrahiertem Gas zurück in das Öl im Entgasungsrezipienten zu vermeiden, wird nun das Ventil geschlossen und dadurch der Gassammelraum vom Entgasungsrezipienten getrennt. Mit dem verschiebbaren Kolben wird nun der Gassammelraum auf sein minimales Volumen verkleinert wird und dadurch das Gas komprimiert und in das Gassammelrohr gedrückt. Das Gassammelrohr wird anschließend über das Ventil vom Gassammelraum getrennt. Anschließend wird der Kolben im Zylinder über den Kolben angetrieben in seine Ausgangsposition bewegt, in der das Volumen des Gassammelraums wieder maximal ist. Das Ventil wird geöffnet, wodurch das Entgasungsrezipient wieder mit dem Gassammelraum verbunden wird, so dass weiteres extrahiertes Gas in den Gassammelraum tritt. Dieses wird auch, wie zuvor baschrieben, komprimiert und auch in das Gassammelrohr transportiert. Dieser Vorgang wird mehrfach wiederholt, bis sich genug extrahiertes Gas im Gassammelrohr angesammelt hat.

Eine andere Gasextraktionsanlage ist aus der US-PS 4,236,404 bekannt. Dort ist ein Entgasungsrezipient vorgesehen, in den Probenöl zur Entgasung eingebracht wird, wobei sich das Gas in dem freien Volumen des Entgasungsrezipienten sammeln kann. Das Einbringen des Probenöls in den Entgasungsrezipienten erfolgt dabei über eine Einfüllvorrichtung, die ein Nadelventil aufweist, durch das der Ölfluss in den Entgasungsrezipienten dosiert wird. Mit einer Magnetrühreinrichtung wird das Probenöl zur Unterstützung der Entgasung gerührt. Nachdem sich genug Gas im freien Volumen des Entgasungsrezipienten angesammelt hat, was anhand des Erreichens eines vorgegebenen Gasdrucks im Entgasungsrezipienten ermittelt wird, wird das Volumen mittels eines im Entgasungsrezipienten vorgesehenen verschiebbaren Kolbens verkleinert; dadurch wird das Gas komprimiert und in ein Sammelrohr gedrückt.

Für die Gas-In-Öl-Analyse (DGA) wird außerdem nach VDE 0370 Teil 9 (identisch mit IEC 567) zur Gasextraktion aus dem Öl eine sogenannte Töpler-Pumpe empfohlen. Bei der bekannten Töpler-Pumpe ist ein unter Unterdruck stehender Entgasungsrezipient vorgesehen, in welchen das gashaltige Öl eingespritzt wird. Das aus dem Öl entweichende Gas tritt dabei aus dem Entgasungsrezipienten in einen Gassammelkolben über. Der Gassammelkolben wird nun mit Quecksilber als Sperrflüssigkeit geflutet, wodurch das Gas durch ein Rückschlagventil in eine über dem Gassammelkolben angeordnete Bürette gedrückt wird. Dieses Verfahren wird mehrere Male wiederholt, bis sich genügend Gas in der Bürette angesammelt hat. Danach wird in einem zweiten Verfahrensabschnitt die Bürette mit einem offenen Quecksilberausgleichsgefäß verbunden. Durch Höhenjustierung des Quecksilberausgleichsgefäßes wird dann das in der Bürette gesammelte Gas auf Atmosphärendruck gebracht. Bei Kenntnis des Atmosphärendrucks und der Raumtemperatur kann anhand des in der Bürette angesammelten Gasvolumens der Gesamtgasgehalt bestimmt werden. Erst im Anschluß an diesen zweiten Verfahrensabschnitt kann das Gas der Analyse zugeführt werden. In nachteiliger Weise ist die Handhabung der Töpler-Pumpe mit einem vergleichsweise hohen Arbeits- und Zeitaufwand verbunden und ist daher entsprechend kostenintensiv. Ein weit gravierenderer Nachteil der Töpler-Pumpe besteht jedoch darin, daß zu deren Betrieb eine vergleichsweise große Menge des hochgiftigen Quecksilbers benötigt wird. Der Betrieb einer herkömmlichen Anlage, die ca. 10 kg Quecksilber enthält, ist daher stets mit einem gewissen Gesundheitsrisiko für das Bedienungspersonal verbunden. Desweiteren ist der Betrieb der Töpler-Pumpe umweltbelastend.

Der Erfindung liegt daher die Aufgabe zugrunde, eine Gasextraktionsanlage der zuerst genannten Art dahingehend zu verbessern, dass unter Beibehaltung des Vorteils der geringen Rückdiffusion eine Gasextraktion für eine Gasanalyse mit geringem Zeitaufwand ermöglicht ist.

Diese Aufgabe wird bei einer Gasextraktionsanlage der erstgenannten Art erfindungsgemäß dadurch gelöst, das durch den verschiebbaren Kolben erreichbare größte Volumen von Gassammelraum und Gasleitung derart bemessen ist, daß darin bei geschlossenenm Ventil eine für eine verläßliche Gasanalyse ausreichende Menge an Gas rückdiffusionsfrei sammelbar ist, und daß die Einfüllvorrichtung mit einem Allin'schen Filterrohr in dem Entgasungsrezipienten endet.

Durch diese großvolumige Gestaltung des Gassammelraums ist darin eine so große Menge Gas sammelbar, dass der verschiebbare Kolben zur Abfuhr des Gases aus dem Gassammelraum in das Analysegerät nur ein einziges Mal betätigt werden muss. Dadurch ist im Vergleich zum Stand der Tachnik eine erhebliche Zeitersparnis erreicht. Eine weitere Zeitersparnis ergibt sich dadurch, dass die Einfüllvorrichtung mit einem Allin'schen Filterrohr in dem Entgasungsrezipienten endet. Das zu entgasende Medium wird über das Allin'sche Filterrohr in den Entgasungsrezipienten gefüllt. Durch das Allin'sche Filterrohr wird das Öl dabei zerstäubt, so dass es als Ölnebel in den Entgasungsrezipienten gelangt. Durch diese Ölnebelbildung tritt das im Öl gelöste Gas besonders rasch aus dem Öl aus und sammelt sich dadurch sehr schnell im Gassammelraum. Die Entgasungszeit ist dadurch erheblich verringert.

Bevorzugt weist die Gasleitung eine Länge auf, die ihren Innendurchmesser um mindestens das 1000-fache übersteigt. Die Gasleitung ist dadurch sehr lang und dünn, so dass eine Rückdiffusion des Gases in das Öl verringert und dadurch auch der durch Rückdiffusion verursachte Messfehler weiter reduziert ist.

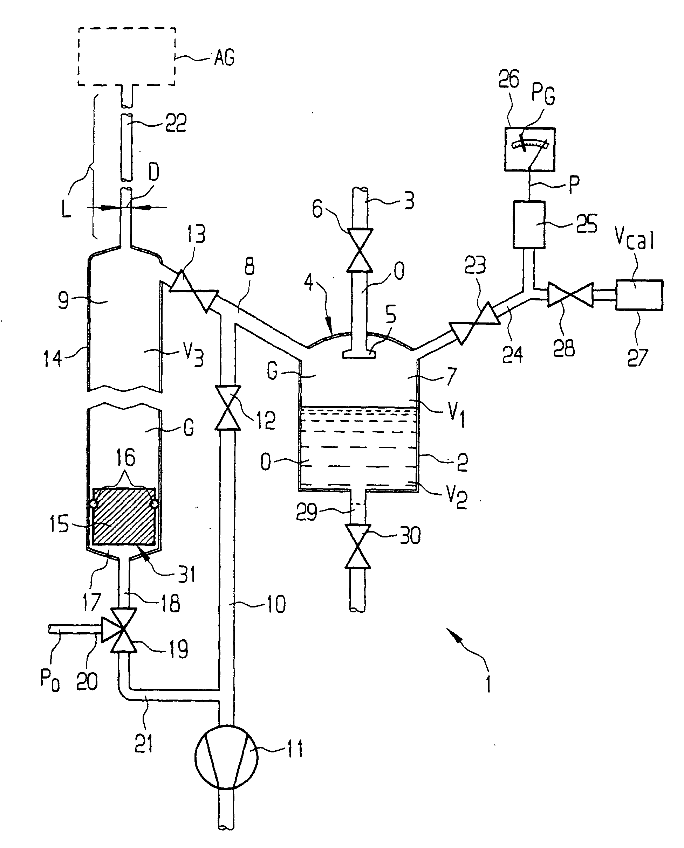

Nachfolgend wird ein Ausführungsbeispiel der Erfindung anhand einer Zeichnung näher erläutert. Dabei zeigt die einzige Figur eine Gasextraktionsanlage mit einem Entgasungsrezipienten und einem mit einem beweglichen Kolben versehenen Gassammelraum.

Die Figur zeigt eine für Öl 0 als zu entgasendes Medium bestimmte Gasextraktionsanlage 1. Die Gasextraktionsanlage 1 umfaßt einen hochvakuumdichten Entgasungsrezipienten 2 aus Metall. Das Öl O wird dabei über eine Zuleitung 3 in den Entgasungsrezipienten 2 eingebracht. Die Zuleitung 3 ist an einer Oberseite 4 des Entgasungsrezipienten 2 in diesen eingeführt und endet innerhalb des Entgasungsrezipienten 2 mit geringem Abstand zu dessen Oberseite 4 in einem sogenannten Allinschen Filterrohr 5. Über dieses tritt das Öl O in den Entgasungsrezipienten 2 aus. Als Allinsches Filterrohr 5 wird üblicherweise eine Glasfritte der Porositätstufe 4 bezeichnet.

Die Zuleitung 3 enthält ein ölfestes Ventil 6, über welches die Ölzufuhr in den Entgasungsrezipienten 2 gesteuert wird. Infolge der ölzufuhr ist der Entgasungsrezipient 2 bei dessen Betrieb teilweise mit Öl O gafüllt. Über dem im Entgasungsrezipienten 2 angesammelten Öl O bildet sich ein Gasraum 7 aus. Das Volumen des Gasraumes 7 wird als freies Gas-volumen V₁ bezeichnet und bestimmt sich aus der Differenz des Rauminhalts des Entgasungsrezipienten 2 und des Ölvolumens V₂ im Entgasungsrezipienten 2. Bei einem Rauminhalt des Entgasungsrezipienten 2 von vorzugsweise 400 ml verbleibt nach dessen Befüllung mit dem zu entgasenden Öl O ein freies Gasvolumen V₁ von typischerweise 200 ml.

Der Gasraum 7 des Entgasungsrezipienten 2 ist über eine Verbindungsleitung 8 mit einem Gassammelraum 9 verbunden. Der Gassammelraum 9 und der Entgasungsrezipient 2 sind über eine von der Verbindungsleitung 8 abzweigende Gasleitung 10 an eine Vakuumpumpe 11 angeschlossen. Die Gasleitung 10 kann dabei mittels eines darin angeordneten Ventils 12 gasdicht gesperrt werden. Ein weiteres Ventil 13 ist in der Verbindungsleitung 8 zwischen dem Gassammelraum 9 und der abzweigenden Gasleitung 10 angeordnet.

Der zylindrisch ausgebildete Gassammelraum 9 wird begrenzt von einem innenseitig geschliffenen Metallbehälter 14, innerhalb dem ein Kolben 15 verschiebbar geführt ist. Der Kolben 15 ist mit einer dessen Umfang umlaufenden Einfräsung versehen, in der ein O-Ring 16 aus Gummi dichtend einliegt. Der Kolben 15 teilt somit den Rauminhalt des Metallbehälters 14 gasdicht in den Gassammelraum 9 und einen diesem bezüglich des Kolbens 15 entgegengesetzten Druckraum 17 auf. Der Druckraum 17 ist an seiner dem Kolben 15 gegenüberliegenden Seite über eine Leitung 18 mit einem Drei-Wege-Ventil 19 verbunden. Das Drei-Wege-Ventil 19 wiederum ist einerseits über eine offene Leitung 20 mit Atmosphärendruck p₀ beaufschlagt und andererseits über eine Vakuumleitung 21 mit der Vakuumpumpe 11 verbunden.

Der Gassammelraum 9 mündet an seiner dem Kolben 15 gegenüberliegenden Seite in eine lange dünne Gasleitung 22, über welche das Gas G einem Analysegerät AG, üblicherweise einem Gas-chromatographen, zugeleitet wird. Die Gasleitung 22 weist dabei eine Länge L von 3,5 m bei einem Innendurchmesser D von 1/8", dies entspricht etwa 1,3 mm, auf. Die Länge L beträgt somit mehr als das 1000-fache des Innendurchmessers D.

Der Gasraum 7 des Entgasungsrezipienten 2 ist über eine mit einem Ventil 23 versehene Leitung 24 mit einem Drucksensor 25 verbunden. Der mit einer Druckanzeige 26 ausgestattete Drucksensor 25 mißt vorzugsweise den absoluten Gasdruck p im Entgasungsrezipienten 2 und - bei geöffnetem Ventil 13 - im Gas-sammelraum 9. Die zusätzliche Messung eines Referenzdrucks (beispielsweise des Atmosphärendrucks p₀) erübrigt sich somit. Zur Kalibrierung der kompletten Anlage (Volumen-Ermittlung) ist diesem ein Gasraum 27 mit einem konstanten Kalibrierungsvolumen V_{cal} parallelgeschaltet. Im Betrieb der Gasextraktionsanlage 1 ist der Gasraum 27 mittels eines Ventils 28 abgesperrt.

Das im Entgasungsrezipienten 2 angesammelte ÖL O kann über einen Ölablaß 29 aus dem Entgasungsrezipienten 2 abgelassen und abgemessen werden. Der Ölablaß 29 ist dazu mit einem ölfesten Ablaßventil 30 versehen. Vorzugsweise ist jedoch der Entgasungsrezipient 2 mit einem Schauglas, das eine kalibrierte Volumenskala zur Ermittlung der Ölmenge enthält, versehen.

Vor Inbetriebnahme der Gasextraktionsanlage 1 wird diese zunächst evakuiert. Dazu sind die Ölventile 6,30 und das Ventil 28 geschlossen. Das Drei-Wege-Ventil 19 verbindet die Leitungen 18 und 21. Die übrigen Ventile 12,13 und 23 sind geöffnet. Der gesamte Innenraum der Gasextraktionsanlage 1 ist somit mit der Vakuumpumpe 11 verbunden und wird mittels dieser auf ein Hochvakuum von etwa 0,050 mbar evakuiert. Der Kolben 15 befindet sich dabei in der dargestellten Stellung. In dieser Stellung des Kolbens 15 weist der Gassammelraum 9 und die Leitung 22 ein maximales Volumen V₃ von vorzugsweise 500 ml auf. Der Gassammelraum 9 ist somit wesentlich größer als das freie Gasvolumen V₁ des Entgasungsrezipienten 2. Nachdem der vom Drucksensor 25 gemessene Druck auf den Wert von 0,050 mbar abgefallen ist, wird das Ventil 12 geschlossen und der Innenraum der Gasextraktionsanlage 1 somit von der Vakuumpumpe 11 abgetrennt.

Die Gasextraktion wird eingeleitet durch Öffnen des Ventiles 6. Das zu entgasende Öl O tritt nun durch das Filterrohr 5 in den Gasrezipienten 2 aus. Infolge des Vakuums im Entgasungsrezipienten 2 entweicht das bisher im Öl O gelöste Gas G in den Gasraum 7. Über die offene Verbindungsleitung 8 tritt das extrahierte Gas G in den Gassammelraum 9 und in Leitung 22 über, wobei stets sowohl im Gassammelraum 9 und Leitung 22 als auch im Gasraum 7 derselbe Gasdruck p herrscht.

Infolge des geringen Druckanstieges (vorzugsweise 6 mbar) wird verhindert, daß vergleichsweise leicht lösliche Gasbestandteile in das sich im Entgasungsrezipienten 2 ansammelnde Öl O rückdiffundieren. Eine solche Rückdiffusion würde das Analyseergebnis verfälschen, zumal vergleichsweise schwerlösliche Gasbestandteile im Gas G überrepräsentiert wären. Aus diesem Grund ist der Gassammelraum 9 und Leitung 22 außerhalb des Entgasungsrezipienten 2 angeordnet und weisen im Vergleich zu dessen Gasraum 7 ein großes Volumen V₃ auf. Eine merkliche Rückdiffusion tritt nur in unmittelbarer Nähe zum Öl O und erst bei steigendem Druck (höher als der bevorzugte Druckanstieg) im Gasraum 7 auf. Der überwiegende Teil des Gases G befindet sich jedoch im Gassammelraum 9 und in der daran anschließenden Gasleitung 22 und bleibt somit hinsichtlich seiner Zusammensetzung unverfälscht.

Infolge des in den Gasraum 7 und den Gassammelraum 9 sowie in Leitung 22 ausgetretenen Gases G steigt der Gasdruck p langsam an. Die Gasextraktion wird beendet, wenn der mittels der Druckanzeige 26 angezeigte Gasdruck p den o.g. bevorzugten Druck p_{G} erreicht hat. So ist sichergestellt, daß sich genügend Gas im Gassammelraum 9 und der Leitung 22 gesammelt hat, damit eine verläßliche Analyse durchgeführt werden kann. Zur Beendigung der Gasextraktion werden gleichzeitig die Ölzufuhr durch Schließen des Ventiles 6 gestoppt und die Verbindungsleitung 8 durch Schließen des Ventiles 13 gesperrt. Der beim Schließen des Ventiles 13 von der Druckanzeige 26 angezeigte Gasdruck p kann in Verbindung mit dem Volumen V₃ des Gassammelraumes 9 und der Leitung 22 zur Berechnung des Gesamtgasgehalts gemäß der eingangs zitierten VDE 0370 Teil 9 herangezogen werden. Ein separater Verfahrensabschnitt zur Bestimmung des Gesamtgasgehalts ist somit eingespart.

Nach Schließen des Ventiles 13 wird über das Drei-Wege-Ventil 19 die Leitung 18 mit der Leitung 20 verbunden und somit belüftet. Auf diese Weise wird die dem Druckraum 17 zugewandte Rückseite 31 des Kolbens 15 mit Atmosphärendruck p₀ beaufschlagt. Der Kolben 15 wird dadurch in Richtung der Leitung 22 verschoben und verkleinert damit den Gassammelraum 9. Das im Gassammelraum 9 und Leitung 22 eingeschlossene Gas G wird somit komprimiert und in komprimierter Form durch die Leitung 22 in das Analysegerät AG gedrückt. Alternativ kann die Leitung 22 durch ein über ein Ventil abnehmbares Gassammelrohr ersetzt werden. Das in dieses Gassammelrohr gedrückte Gas G kann dann im Gassammelrohr gelagert oder zur externen Analyse transportiert werden.

Die Gasextraktionsanlage 1 genügt allen in der VDE 0370 Teil 9 gestellten Anforderungen. Vergleichsmessungen haben gezeigt, daß die Gasextraktionsanlage 1 sowohl hinsichtlich der Absolutwerte als auch hinsichtlich der Empfindlichkeit einer Gas-In-Öl-Analyse einer Töpler-Pumpe gemäß VDE 0370 Teil 9 entspricht.

Zur Wiederherstellung des Ausgangszustandes der Gasextraktionsanlage 1 wird über das Drei-Wege-Ventil 19 der Druckraum 17 erneut an die Vakuumpumpe 11 angeschlossen. Durch den auf die Rückseite 31 des Kolbens 15 wirkenden Unterdruck wird der Kolben 15 wieder in die dargestellte Ausgangsposition gezogen. Danach wird durch Öffnen des Ablaßventils 30 das Öl O aus dem Entgasungsrezipienten 2 entfernt und dieser gegebenenfalls gereinigt. Die Gasextraktionsanlage 1 kann nun zu einer weiteren Messung erneut evakuiert werden.

## Patentansprüche

1. Gasextraktionsanlage, insbesondere für eine Gas-In-Öl-Analyse, mit einem Entgasungsrezipienten (2), der eine Einfüllvorrichtung (5) für ein zu entgasendes Medium (O) aufweist und der über ein Ventil (13) mit einem außerhalb des Entgasungsrezipienten (2) angeordneten zylindrisch ausgebildeten Gassammelraum (9) verbindbar ist, dessen Volumen mittels eines verschiebbaren Kolbens (15) veränderbar ist und der über eine Gasleitung (22) mit einem Analysegerät (AG) verbunden ist,
**dadurch gekennzeichnet, daß** das durch den verschiebbaren Kolben (15) erreichbare größte Volumen (V₃) von Gassammelraum (9) und Gasleitung (22) derart bemessen ist, daß darin bei geöffnetem Ventil (13) eine für eine verläßliche Gasanalyse ausreichende Menge an Gas rückdiffusionsfrei sammelbar ist, und daß die Einfüllvorrichtung (5) mit einem Allin'schen Filterrohr (5) in dem Entgasungsrezipienten (2) endet.

2. Gasextraktionsanlage nach Anspruch 1,
**dadurch gekennzeichnet, daß** die Gasleitung (22) eine Länge (L) aufweist, die ihren Innendurchmesser (D) um mindestens das 1000-fache übersteigt.

## Claims

1. Gas extraction facility, in particular for a gas-in-oil analysis, having a degassing receptacle (2), which has a filling device (5) for a medium (O) which is to be degassed and which can be connected, via a valve (13), to a gas-collection space (9) which is arranged outside the degassing receptacle (2), is of cylindrical design, the volume of which can be varied by means of a displaceable piston (15) and which is connected to an analysis appliance (AG) via a gas line (22), **characterized in that** the maximum volume (V₃) of gas-collection space (9) and gas line (22) which can be achieved using the displaceable piston (15) is dimensioned in such a manner that, in this volume, when the valve (13) is open, it is possible to collect, without back-diffusion, a quantity of gas which is sufficient for reliable gas analysis, and **in that** the filling device (5) ends with an allihn filter tube (5) in the degassing receptacle (2).

2. Gas extraction facility according to Claim 1, **characterized in that** the length (L) of the gas line (22) exceeds its internal diameter (D) by at least 1000 times.

## Revendications

1. Installation d'extraction des gaz, en particulier pour une analyse gaz dans huile, comportant un récipient de dégazage (2), qui comporte un dispositif de remplissage (5) pour un fluide (O) à dégazer et qui par l'intermédiaire d'une vanne (13) peut être mis en communication avec un espace collecteur de gaz (9), de forme cylindrique et disposé à l'extérieur du récipient de dégazage (2), espace collecteur de gaz, dont le volume peut être modifié par l'intermédiaire d'un piston coulissant (15) et qui par l'intermédiaire d'une conduite de gaz (22) communique avec un appareil d'analyse (AG), **caractérisée en ce que** le volume maximal (V₃) de l'espace collecteur de gaz (9) et de la conduite de gaz (22), pouvant être réalisé par le piston coulissant (15), est défini de telle sorte que, la vanne (13) étant ouverte, une quantité de gaz suffisante pour permettre une analyse fiable du gaz puisse s'y accumuler sans rétrodiffusion, et que le dispositif de remplissage (5) débouche dans le récipient de dégazage (2) par un tube filtrant d'Allin (5).

2. Installation d'extraction des gaz selon la revendication 1, **caractérisée en ce que** la conduite de gaz (22) a une longueur (L) qui est au moins 1000 fois supérieure à son diamètre intérieur (D).
